**Europäisches Patentamt**

(19) **European Patent Office**

**Office européen des brevets**

(11) Veröffentlichungsnummer: **0 121 281**
**B1**

(12) **EUROPÄISCHE PATENTSCHRIFT**

(45) Veröffentlichungstag der Patentschrift:
23.09.87

(51) Int. Cl.⁴: **G 11 B 15/44,** G 11 B 15/02

(21) Anmeldenummer: **84200446.7**

(22) Anmeldetag: **28.03.84**

(54) Vorrichtung an dem reversierenden Laufwerk eines Magnetbandkassettengerätes.

(30) Priorität: 02.04.83 DE 3312134
11.04.83 NL 8301265

(43) Veröffentlichungstag der Anmeldung:
10.10.84 Patentblatt 84/41

(45) Bekanntmachung des Hinweises auf die Patenterteilung:
23.09.87 Patentblatt 87/39

(84) Benannte Vertragsstaaten:
CH DE FR GB IT LI SE

(56) Entgegenhaltungen:
EP-A-0 095 815
GB-A-2 018 001
US-A-3 533 633
US-A-3 759 464
US-A-3 870 248
US-A-4 159 492
US-A-4 283 027

(73) Patentinhaber: **Philips Patentverwaltung GmbH,
Wendenstrasse 35 Postfach 10 51 49, D-2000
Hamburg 1 (DE)**
(84) Benannte Vertragsstaaten: **DE**

(73) Patentinhaber: **N.V. Philips' Gloeilampenfabrieken,
Groenewoudseweg 1, NL- 5621 BA Eindhoven (NL)**
(84) Benannte Vertragsstaaten: **CH FR GB IT LI SE**

(72) Erfinder: **Deutsch, Armin, Berliner Ring 109, D-6330
Wetzlar (DE)**
Erfinder: **Ruyten, Henricus M., Volpertshäuser
Strasse 14, D-6330 Wetzlar (DE)**

(74) Vertreter: **Kupfermann, Fritz- Joachim, Dipl.- Ing.,
Philips Patentverwaltung GmbH Wendenstrasse
35 Postfach 10 51 49, D-2000 Hamburg 1 (DE)**

## Beschreibung

Die Erfindung bezieht sich auf eine Vorrichtung an dem reversierenden Laufwerk eines Magnetbandkassettengerätes, bei dem das Magnetband an wenigstens einem Aufnahme- bzw. Wiedergabetonkopf vorbeigefürt wird, der an einer als Schwinge ausgebildeten, gegen das Magnetband verfahrbaren Kopfplatte angeordnet ist, die infolge eines gesteuerten Verschwenkens immer eine von zwei an ihr ageordneten Andruckrollen gegen die zugehörige Tonwelle überführt und damit die Bandlaufrichtung bestimmt.

Eine derartige Vorrichtung ist aus dem Laufwerk der Fa. Grundig WKC 3 3857 VD bekannt. Das genannte Gerät arbeitet mit einem reversierenden Motor und Antrieb, der Steuerteller dreht, die mit Nocken versehen sind. Entsprechend der Stellung dieser Nocken auf den Steuertellern kann die Kopfplatte bei ihrem Verfahren auf die Kassette gegen die verschieden weit von der Kassettenvorderwand entfernten Nocken stoßn, so daß die jeweilige, den Bandtransport übernehmende Tonrolle gegen das Magnetband gedrückt wird.

Der Einsatz eines reversierbaren Antriebs für das Steuern der Kopfplatte ist aufwendiger als der Einsats eines Antriebs mit nur einer Laufrichtung. Beim Einsatz eines nur in einer Laufrichtung arbeitenden Motors läßt sich der bekannte Schwenkmechanismus nicht verwirklichen.

Es ist Aufgabe der Erfindung, eine Vorrichtung an dem reversierenden Laufwerk eines Magnetbandgerätes zu schaffen, mit dem die Schwenklage der Kopfplatte jedesmal von Neuem bestimmt wird von ihr selbst bei ihrem Vorfahren in Richtung auf das Magnetband.

Die gestellte Aufgabe ist erfindungsgemäß gelöst durch
- am Laufwerkschassis vorgesehene, beiderseits des Tonkopfes angeordnete Leitschlitzanordnungen,
- an der Kopfplatte vorgesehene Leitstifte, die in den Leitschlitzanordnungen auf die gedachte Tonwellen-Verbindungslinie zufahrem können,
- mit den Leitschlitzanordnungen zusammenwirkende Steuermittel, die die Leitstifte in den Leitschlitzanordnungen derart lenken, daß die Leitstifte nach jedem Zurückfahren der Kopfplatte von der Verbindungslinie beim erneuten Vorfahren auf die Verbindungslinie wechselweise in der einen oder anderen Leitschlitzanordnung in einen Leitschlitzschenkel einfahren, der dem betreffenden Leitstift den Vorfahrweg blockiert, wodurch sich jeweils die Andruckrolle der verschwenkenden Kopfplatte, deren zugeordneter Leitstift einen unblockierten Vorfahrweg vorfindet, zur zugeordneten Tonwelle vorfahren kann.

Der wesentliche Vorteil dieser Vorrichtung besteht darin, daß die Kopfplatte selbst bei ihrem Zurückfahren von der Kassette und erneuten Vorfahren in Richtung auf die Kassette nur auf mechanische Weise Anschläge bildet, die durch das Zusammenarbeiten der Leitstifte mit Teilen der Leitschlitzanordnung gebildet werden. Die Steuermittel sorgen auf mechanische Weise dafür, daß der Anschlag zuverlässig von der einen Seite auf die andere Seite wechselt und damit wechselweise immer nur eine Andruckrolle der Kopfplatte gegen die zugeordnete Tonwelle drücken kann. Ein weiterer Vorteil dieser Vorrichtung besteht darin, daß das Verstellen der Kopfträgerplatte bzw. das wechselweise Anlegen der Andruckrollen an die Tonwellen mit einem nur in einer Richtung umlaufenden Antrieb erfolgen kann, wodurch das Antrieb wirtschaftlicher herstellbar ist.

Nach einer weiteren Ausgestaltung der Erfindung ist vorgesehen, daß die Leitschlitzanordnungen aus am Laufwerkchassis vorgesehenen gabelförmigen Leitschlitzen mit zwei den Gabelzinken entsprechenden Schenkeln bestehen, von denen einer kürzer als der andere ausgebildet ist und die kürzeren (bzw. längeren) Schenkel beide entweder aufeinander zu oder voneinander weg angeordnet sind, und daß sich ein dem Gabelschaft entsprechender mittiger Einlaufschlitz an den trichterförmigen Gabelfuß anschließt. Weiterhin ist vorgesehen, daß die Steuermitel aus einer Steuerplatte bestehen, die parallel zur Tonwellen-Verbindungslinie verschiebbar und beiderseits des Tonkopfes mit U-förmigen Leitschlitzen versehen ist, deren Schenkel mit den Schenkeln der zugeordneten gabelförmigen Leitschlitze überdeckbar sind, jedoch einen geringeren gegenseitigen Abstand voneinander aufweisen, wodurch zum wechselweisen Positionieren der Leitstifte die kürzeren oder längeren Schenkel die angesptzten freien Enden der Materialzungen zwischen den Schenkeln der U-förmigen Leitschlitze - in den Steuerplattenendstellungen und bei zurückgefahrener Kopfplatte - wechselweise in der einen oder anderen Verschieberichtung seitlich gegenüber den Einlaufschlitzen versetzbar sind.

Das einzige, für die Schwenkrichtung der Kopfplatte entscheidende Bauteil ist damit die Steuerplatte, die aus einem flachen Stanzteil mit nur geringer Bauhöhe bestehen kann. Damit ist dieser Aufbau außerordentlich geeignet für flachbauende Geräte, wie z. B. Autokassettenspieler. Die Leitstifte können beispielsweise auf einfache Weise nach Art von Reißbrettstiften oder als elastische Zungen mit am freien Ende angeordneten Stiftansätzen ausgebildet sein. Der geringere gegenseitige Abstand der Schenkel der U-förmigen Schlitze in der Steuerplatte sorgt dafür, daß die Leitstifte, die bei einem Zurückfahren der Kopfplatte in die mittigen Einlaufschlitze eingelaufen sind, die Zungen der Steuerplatte immer seitlich versetzt vorfinden, wodurch eine sichere Steuerung in der gewünschten Richtung erreicht wird.

Bei einer anderen Ausführungsform der Leitschlitzanordnungen und der Steuermittel ist vorgesehen, daß die Leitschlitzanordnungen aus am Laufwerkschassis vorgesehenen U-förmigen Rinnen mit jeweils einem kürzeren und einem längeren Schenkel und einer Doppelrinne an der die Schenkel verbindenden Basis versehen sind und daß die Steuermittel als erhabene Führungsflächen am Rinnenboden und Führungskonturen im Bereich des Rinnenbodens ausgebildet sind, die es den Leitstiften nach jedem Zurückfahren mit der Kopfplatte und beim erneuten Vorfahren nur gestatten, über eine der Basisrinnen von dem kürzeren der Schenkel in den langeren Schenkel und beim nachfolgenden Zurück- und Vorfahren über die andere Basisrinne von dem längeren in den kürzeren Schenkel einzulaufen, wobei die kürzeren und längeren Schenkel so verteilt sind, daß immer bei einem Zurück- und Vorfahren einer der Leitstifte in einen kürzeren Schenkel einläuft, während der andere Leitstift in den längeren Schenkel einläuft und beim darauffolgenden Zurück- und Vorfahren dieser Leitstift in den längeren und der andere in den kürzeren Schenkel einläuft.

Bei diesen Leitschlitzanodnung und Steuermitteln wird überhaupt kein bewegliches Steuermittel benötigt. Das Steuermittel in Form von erhabenen Führungsflächen und Führungskonturen kann im Spritzgußverfahren, z. B. dem Outsert-Molding, an der Chassisplatte einstückig gebildet werden. Das einzige bewegliche Element sind die Leitstifte der Kopfplatte.

Nach einer weiteren Ausgestaltung der Erfindung ist vorgesehen, daß die Steuerplatte mit wenigstens einem Anschlag versehen ist und daß das Gerät eine Stoptaste und eine damit verbundene Stange aufweist, die mit einer Nase versehen ist, die beim Bedienen der Stoptaste gegen den Anschlag stößt und die Steuerplatte immer in die eine ihrer Endstellungen verschiebt. Damit entsteht eine Vorzugslage der Steuerplatte, die zur Folge hat, daß das Magnetband nach Bedienung der Stoptaste immer nur in der einen der zwei Transportrichtungen transportiert wird. Die Stoptaste kann bei einem Autokassenspieler gleichzeitig die Auswurftaste sein, wodurch erreicht wird, daß beim Eindrücken der Auswurftaste, dem Entnehmen der gespielten Kassette und dem Einsetzen einer neuen Kassette sicher ist, daß das Band in einer bestimmten Richtung transportiert wird.

Die Erfindung wird anhand des in der Zeichnung dargestellten Ausführungsbeispieles näher erläutert. Es zeigen:

Fig. 1 eine ansicht des Magnetbandkassettengerätes von der Antriebsseite her,

Fig. 2A eine schaubildlliche vergrößerte Darstellung eines Rastmechanismus des Gerätes in einer ersten Lage,

Fig. 2B den Rastmechanismus nach Fig. 2A in einer zweiten Lage,

Fig. 3 das Magnetbandkassettengerät von der Tonkopfseite her gesehen in einer Spielstellung einer ersten Bandlaufrichtung,

Fig. 4 das Magnetbandkassettengerät von der Tonkopfseite her gesehen in einer zu Fig. 3 entgegengesetzten Bandrichtung,

Fig. 5 das Zusammenwirken einer Leitschlitzanordnung mit Steuermitteln und Kopfplatte in einer Explosionsdarstellung,

Fig. 6 die verschiedenen Lagen einer Steuerplatte zur Steuerung der Leitschlitzschlitzanordnung und der Kopfplatte,

Fig. 7 eine schaubildliche Darstellung von Steuerplatte und mit ihr zusamenwirkender Stoptaste zur Vorgabe einer Vorzugsbandtransportrichtung,

Fig. 8 eine zweite Ausführungsform der Leitschlitzanordnung und der Steuermittel für das Verschwenken der Kopfplatte.

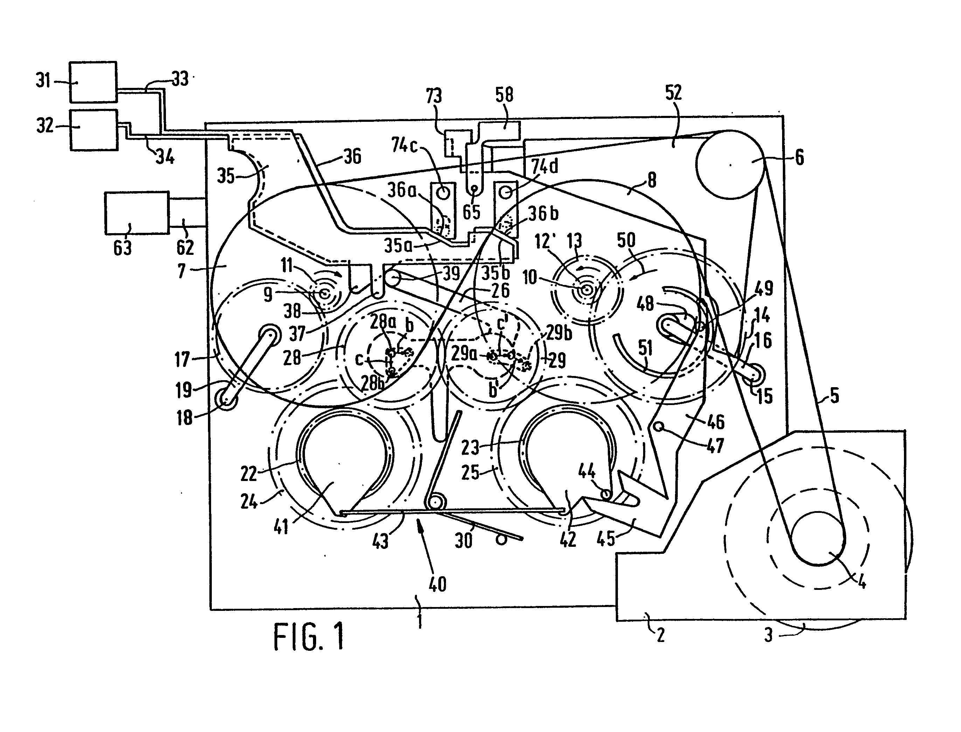

Das Magnetbandkassettengerät nach der Erfindung, wie in Fig. 1 dargestellt, weist ein Gestell 1 auf, das über eine Stütze 2 einen nur in einer Richtung umlaufenden Antriebsmotor 3 trägt. Das Abriebsritzel 4 des Motors 3 führt eine Peese 5, die über eine Umlenkrolle 6 über Schwungräder 7 und 8 derart geführt ist, daß die Schwungräder gegensinnig drehen. Die Schwungräder 7 und 8 sind im Gestell 1 gelagert. Die Schwungscheibe 7 ist fest verbunden mit einer Tonwelle 9, und die Schwungscheibe 8 ist fest verbunden mit einer Tonwelle 10. Weiterhin ist die Schwungscheibe 7 mit einem Zahnrad 11 verbunden; und die Schwungscheibe 8 ist mit einem Zahnrad 12 verbunden. Über eine Rutschkupplung ist koaxial zu dem Zahnrad 12 ein weiteres Zahnrad 13 auf der Schwungscheibe 8 drehbar angeordnet. Mit dem Zahnrad 12 auf der Schwungscheibe 8 ist ständig kämmend ein als Zahnrad ausgebildetes Schaltrad 14 in Einriff. Dieses Zahnrad 14 ist schwenkbar um eine Achse 15. Die Achse 15 trägt einen in Fig. 1 schematisch dargestellten Schwenkarm 16, auf dem drehbar das Schaltrad 14 gelagert ist. In ähnlicher Weise kämmt mit dem Zahnrad 11 ständig ein Zahnrad 17. Dieses Zahnrad 17 ist drehbar um eine Schwenkachse 18 mittels eines schematisch dargestellten Schwenkarmes 19.

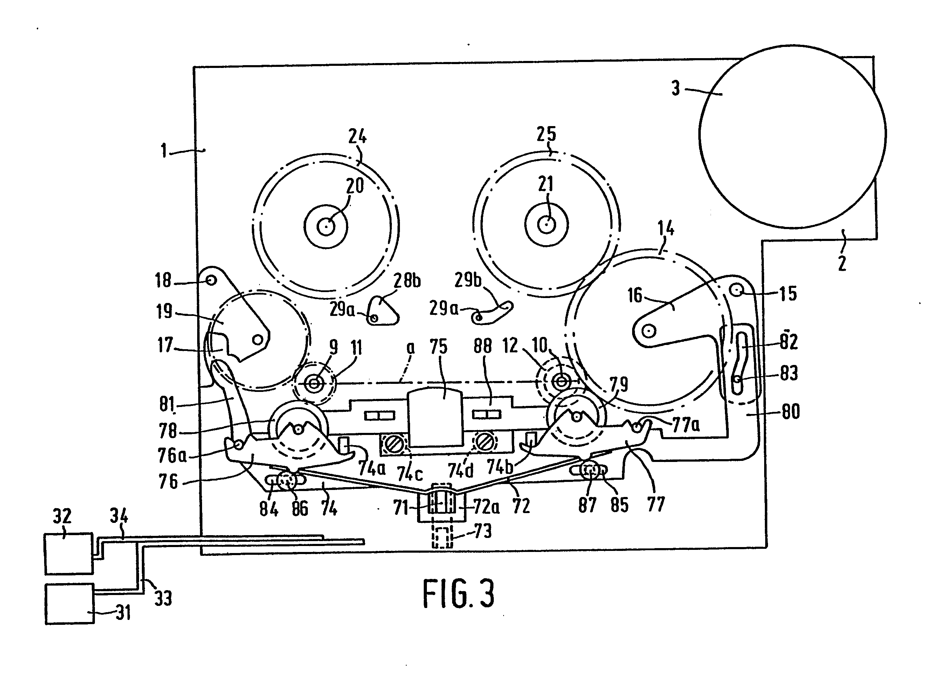

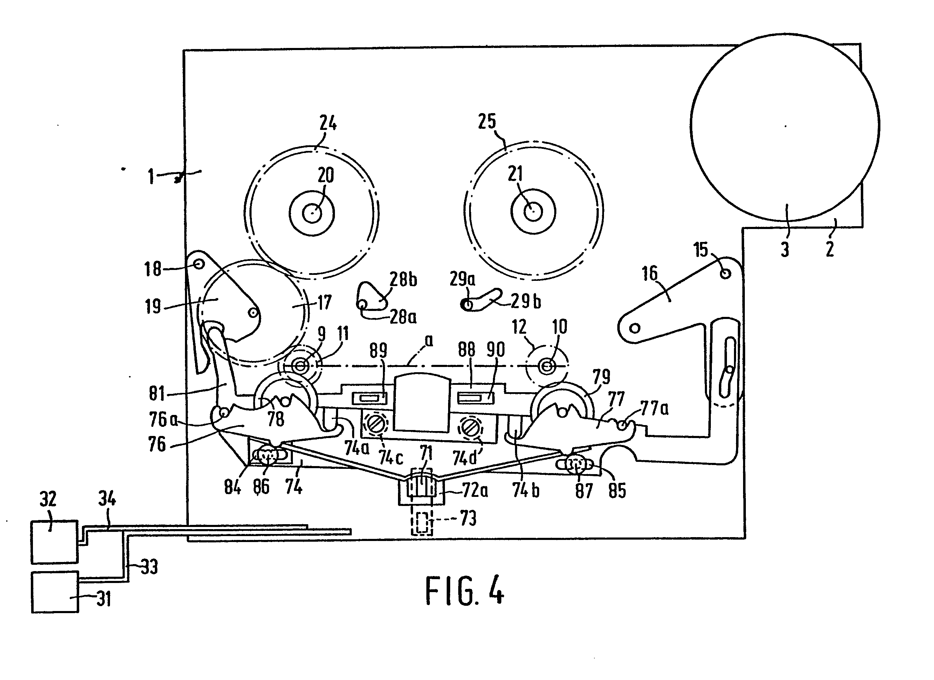

Wie auch in Fig. 3 und 4 dargestellt, sind drehbar im Gestell 1 Wickeldorne 20 und 21 gelagert. Diese Wickeldorne 20 und 21 sind fest verbunden mit Schnellspulrädern 22, 23. Über Rutschkupplungen sind mit den Wickeldornen 20, 21 Spielräder 24, 25 verbunden.

In Fig. 3 ist dargestellt, wie das Spielrad 25 über das Schaltrad 14 von dem Zahnrad 12 angetrieben wird. In Fig. 4 ist auf ähnliche Weise zu sehen, wie das Spielrad 24 über das Zahnrad 17 vom Zahnrad 11 angetrieben wird.

Ein Bügel 26, der verschiebbar im Gestell gelagert ist, trägt nebeneinander zwei Zahnräder 28, 29 (siehe Fig. 1). Eine Feder 30 drückt den Bügel 26 immer in eine in Fig. 1 dargestellte Ausgangsposition.

Der Bügel 26 trägt zwei Stifte 28a und 29a, die in einem dreieckigen Loch 28b bzw. einem Schlitz 29b verschiebbar sind im Gestell 1.

Zum schnellen Vor- und Rücklauf sind zwei Tasten 31 und 32 vorgesehen. Über Tastenstangen 33 und 34 sind diese Tasten 31, 32 mit Schiebern 35, 36 verbunden. Diese Schieber 35 und 36 wirken auf den Bügel 26 ein. Dazu ist der Schieber 35 mit einem Ansatz 37 versehen, der Schieber 36 trägt entsprechend eine Auflauffläche 38. Der Ansatz 37 und die Auflauffläche 38 arbeiten auf einen Stift 39 des Bügels 26. Weiter sind die Schieber 35, 36 mit Auflaufflächen 35a, 35b bzw. 36a, 36b versehen, die arbeiten können auf Stifte 74c, 74d einer Kopfplatte 74 (Fig. 1, 3, 4).

Wenn durch Eindrücken der Schnellspultaste 31 der Ansatz 37 gegen den Stift 39 des Bügels 26 drückt, kommt erst das Zahnrad 29 in Eingriff mit dem Zahnrad 13. Dabei macht der Stift 28a eine Bewegung in Richtung des Pfeiles b und der Stift 29a eine Bewegung in Richtung des Pfeiles c'. Bei weiterer Bewegung fährt der Stift 29a weiter in Richtung des Pfeiles b' und kommt das Zahnrad 29 auch in Eingriff mit dem Zahnrad 23. Das Magnetband wird jetzt mit dem Wickeldorn 21 schnell aufgewickelt. Wird die andere Schnellspultaste 32 eingedrückt, dann wirkt die Auflauffläche 38 auf den Stift 39 des Bügels 26 ein, wodurch der Stift 28a in Richtung des Pfeiles c und der Stift 29a in Richtung des Pfeiles c' fährt, wodurch das Zahnrad 29 mit dem Zahnrad 13 in Eingriff und das Zahnrad 28 mit dem Zahnrad 22 in Eingriff kommt. Dadurch wird der andere Wickeldorn 21 zum Schnellspulen angetrieben.

Wenn die Kopfplatte 74 in eine Spielstellung gelangt ist, nehmen die Stifte 74c, 74d eine der gestrichelten Lagen in Fig. 1 ein. Die Lagen stimmen überein mit den Lagen gemäß Fig. 3 bzw. Fig. 4. Das Eindrücken der Bedienungsstange 33 bzw. 34 hat jetzt zur Folge, daß über die Auflauffläche 35a, 35b bzw. 36a, 36b die Stifte 74c, 74d und damit die Kopfplatte 74 nach links geschoben werden, so daß ein Tonkopf 75 und Andruckrollen 78, 79 abgehoben werden von dem Magnetband.

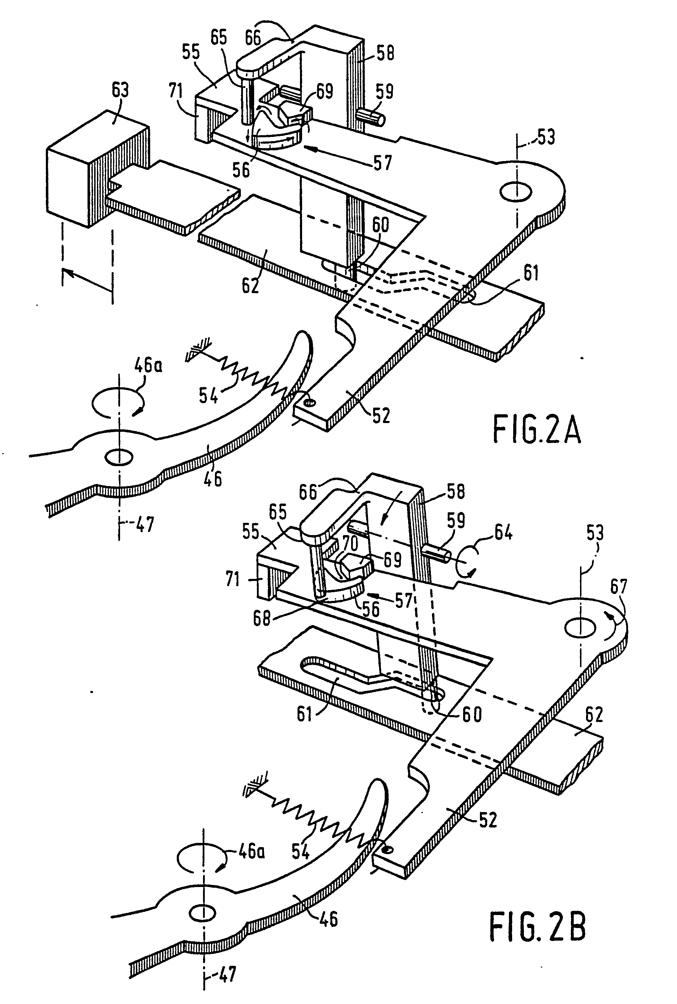

Ein Detektionsorgan 40, bestehend aus zwei Scheiben 41, 42 und einer Verbindungsstange 43, ist über Rutschkupplungen mit den Wickeldornen 20, 21 verbunden. Auf der Scheibe 42 ist ein Stift 44 angeordnet, der innerhalb einer Schaltgabel 45 liegt. Die Schaltgabel 45 ist an einem Schaltelement 46 angeordnet, die um ein Lager 47 drehbar ist (vergl. auch Fig. 2A und 2B). Das Schaltrad 14 trägt in seiner Mitte erhaben vorstehend einen ein wenig ellipsenförmigen Nocken 48. Das Schaltelement 46 greift über das Schaltrad 14 und trägt einen Stift 49, der bei nach innen geschwenktem Schaltelement 46 um den Nocken 48 herumfahren kann bei Drehung des Schaltrades in Richtung eines Pfeiles 50. Um den Nocken 48 herum erstreckt sich in einem größeren Abstand eine erhaben aus dem Schaltrad 14 herausstehende, spiralförmige Steuerkurve 51. Auch diese Steuerkurve 51 kann mit dem Stift 49 an dem Schaltelement 46 zusammenwirken, und zwar immer dann, wenn das Detektionsorgan den Bandstillstand feststellt.

In diesem Fall drückt der Stift 44 nicht mehr gegen die Schaltgabel 45, wodurch der Stift 49 am Schaltelement 46 nicht mehr nach innen in Richtung auf den Nocken 48 belastet wird, sondern stehen bleibt und bei fortgehender Drehung des Schaltrades 14 in Richtung des Pfeiles 50 auf die Außenseite der Steuerkurve 51 gelangt. Weil die Steuerkurve 51 spiralförmig ist, wird das Schaltelement 46 im Uhrzeigersinn um sein Lager 47 verschwenkt, wodurch das Schaltelement 46 gegen ein Verbindungselement 52 gedrückt wird.

Das Verbindungselement 52 ist ein doppelarmiger Hebel, der um ein Lager 53 schwenkbar ist. Wie aus den schaubildlichen Darstellungen in Fig. 2A und 2B zu erkennen ist, ist das Verbindungselement 52 aufgrund der Wirkung einer Feder 54 im Uhrzeigersinn federbelastet. Damit spannt die Bewegung des Schaltelementes 46 die Feder 54.

Der von dem Angriffspunkt des Schaltelementes 46 abgewandte Hebelarm 55 des Verbindungselementes 52 trägt eine Herzkurve 56, die zu einem Rastmechanismus 57 gehört. Zu dem Rastmechanismus 57 gehört auch ein Rasthebel 58, der um eine Achse 59 verschwenkbar ist. Die Achse 59 liegt parallel zu der Oberfläche des Gestelles 1 und zu einer gedachten Verbindungslinie a zwischen den Tonwellen 9 und 10. Der Rasthebel 58 trägt auf der zur Herzkurve 56 gerichteten Seite einen Führungsstift 60, der in einem Führungsschlitz 61 in einer Stange 62 einer Stop/Auswurftaste 63 vorgesehen ist. Der Führungsschlitz 61 hat einen solchen Verlauf, daß der Rasthebel 58, wie sich aus Fig. 2B ergibt, bei ausgeschobener Stop/Auswurftaste um die Achse 59 in Richtung eines Pfeiles 64 verschwenkt wird. Das Nach-Außen-Schieben der Stop/Auswurftaste 63 bzw. der Stange 62 erfolgt auf nicht dargestellte Weise durch das Einschieben einer Magnetbandkassette in das Gerät. Fig. 2A zeigt die Stange 62 in der eingeschobenen Stellung, während die Fig. 2B die Stange 62 in ausgeschobener Stellung zeigt.

Der Rasthebel 58 trägt einen Raststift 65, der in die Herzkurve 56 einfahrbar ist und die Herzkurve umfahren kann. Die Umfahrbarkeit wird z. B. über ein Filmgelenk 66 herbeigeführt. In Fig. 2A liegt der Stift 65 oberhalb der Herzkurve 56, da die Stange 62 eingedrückt ist. In Fig. 2B, wo die Stange 62 ausgefahren ist, ist der Stift 65 in den Bereich der Herzkurve 56 hineingefahren. Damit kann der Stift 65 mit der Herzkurve 56 zusammenwirken, wenn das Schaltelement 46 das Verbindungselement 52, wie in der Darstellung nach Fig. 2B, entgegen dem Uhrzeigersinn in Richtung eines Pfeiles 67 verschwenkt wird. Bei diesem Verschwenken ist der Stift 65 an einer Seitenwand 68 der Herzkurve 56 entlang gelaufen. Ein Führungsnocken 69 sorgt dafür, daß der Stift 65 in eine Mulde 70 der Herzkurve 56 einfällt.

Am Hebelarm 55 ist ein Nocken 71 angeordnet. Dieser Nocken 71 wirkt, wie sich aus den Fig. 3 und 4 ergibt, mit einer Blattfeder 72 zusammen und erstreckt sich durch einen Längsschlitz 23 im Gestell 1 hindurch. Die Blattfeder 72 drückt mit ihren freien Enden gegen Halterungen 76, 77 die auf einer Kopfplatte 74 gelagert sind. Diese Kopfplatte 74 trägt in ihrer Mitte einen Tonkopf 75 und beiderseits des Tonkopfes 75 die Halterungen 76, 77, in denen Andruckrollen 78, 79 gelagert sind. Die Halterungen werden gegen Anschläge 74a, 74b der Kopfplatte 74 gedrückt.

Die Kopfplatte 74 hat abgewinkelte Enden 30, 81. Das abgewinkelte Ende 80 weist einen Schlitz 82 auf, in dem ein Stift 83 verschiebbar ist, der auf dem Schwenkarm 16 befestigt ist. Dadurch ist in der Stellung nach Fig. 3 der Schwenkarm 16 derart um die Achse 15 verschwenkt, daß das Schaltrad 14 kämmt mit dem Spielrad 25 und dem Zahnrad 12. Dementsprechend ist in Fig. 4 dargestellt, daß das abgewinkelte Ende 81 der Kopfplatte 74 den Schwenkarm 19 um die Schwenkachse 18 geschwenkt hat, wodurch das Zahnrad 17 mit dem Spielrad 24 und dem Zahnrad 11 kämmt. Dementsprechend ist in der Stellung nach Fig. 3 die Druckrolle 79 gegen die Tonwelle 10 gedrückt, während in der Stellung nach Fig. 4 die Andruckrolle 78 gegen die Tonwelle 9 gedrückt ist.

In der Kopfplatte 74 sind parallel zu der Verbindungslinie zwischen den Tonwellen 9, 10 Längsschlitze 84, 85 angeordnet, in denen Leitstifte 86, 87 verschiebbar sind.

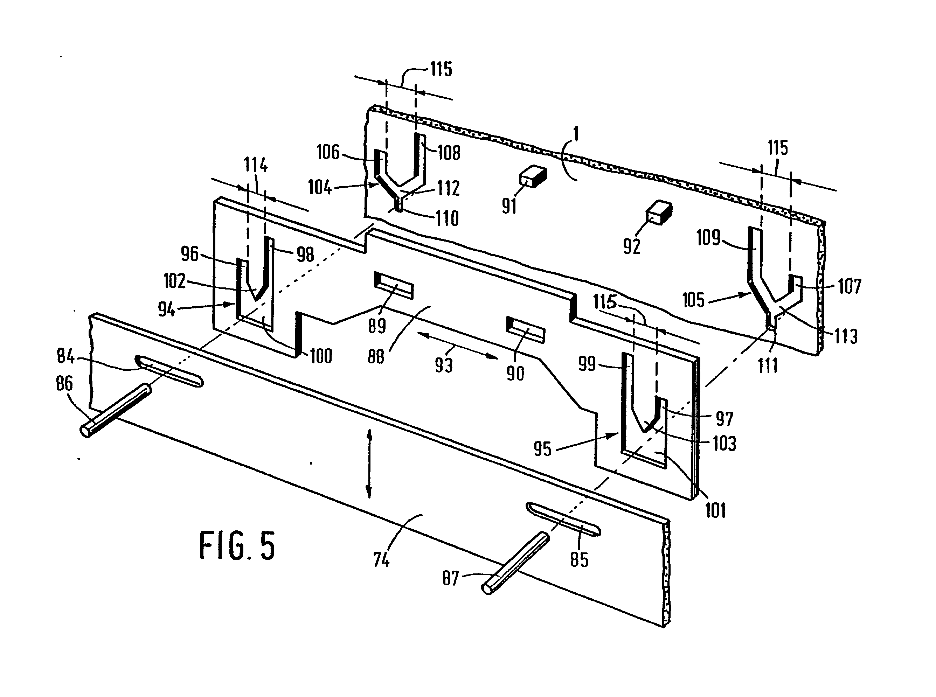

Fig. 5 zeigt anhand der Explosionsansicht in der Kopfplatte 74 geführte Leitstifte 86, 87, die in den Längsschlitzen 84, 85 geführt sind. Die Leitstifte sind damit parallel zu der Verbindungslinie a zwischen den Tonwellen verschiebbar. Die Halterung der Leitstifte 86, 87 an der Kopfplatte 74 ist in den Zeichnungen nicht näher dargestellt. Bei einer einfachen Ausführungsform sind die Leitstifte wie die Stifte von Reißnägeln an einer kappenförmigen Platte befestigt. Es ist aber ebensogut auch möglich, die Leitstifte an den freien Enden von federnden Zungen anzuordnen, die an der Kopfplatte 74 befestigt sind.

Zwischen der Kopfplatte 74 und der Chassisplatte an der Oberseite des Gestells 1 ist eine als Steuermittel dienende Steuerplatte 88 angeordnet. Diese Steuerplatte 88 ist mit Längsschlitzen 89, 90 versehen, durch die Führungsnocken 91, 92 der Chassisplatte hindurchgreifen. Dadurch ist die Steuerplatte 88 parallel zu der gedachten Verbindungslinie zwischen den Tonwellen 9, 10 verschiebbar. Die Verschiebbarkeit ist in Fig. 5 durch einen Doppelpfeil 93 angegeben.

Die Steuerplatte 88 ist an beiden Enden mit U-förmigen Schlitzen 94, 95 versehen. Von den U-förmigen Schlitzen 94 und 95 ist jeweils ein Schenkel 96, 97 kürzer und ein Schenkel 98, 99 länger ausgebildet. Die kürzeren Schenkel sind voneinander weg angeordnet und die längeren Schenkel dementsprechend aufeinander zu. Die Verbindungsbasis 100 und 101 zwischen den kürzeren und längeren Schenkeln ist stark verbreitert ausgebildet. Zwischen den längeren und kürzeren Schenkeln sind Materialzungen 102 und 103 ausgebildet, die an ihren zur Basis 100, 101 gerichteten Enden angespitzt sind.

In der Chassisplatte des Gestells 1 sind als Leitschlitzanordnungen dienende gabelförmige Leitschlitze 104 und 105 angeordnet mit zwei den Gabelzinken entsprechend Schenkeln, von denen jeweils einer 106, 107 kürzer als der andere 108, 109 ausgebildet sind. Am Gabelschaft ist ein mittiger Einlaufschlitz 110, 111 vorgesehen, der sich an den trichterförmigen Gabelfuß 112, 113 anschließt. Der gegenseitige Abstand 114 der Schenkel der U-förmigen Leitschlitze 94, 95 in der Steuerplatte 88 ist geringer als der gegenseitige Abstand 115 der Schenkel der gabelförmigen Leitschlitze 104, 105.

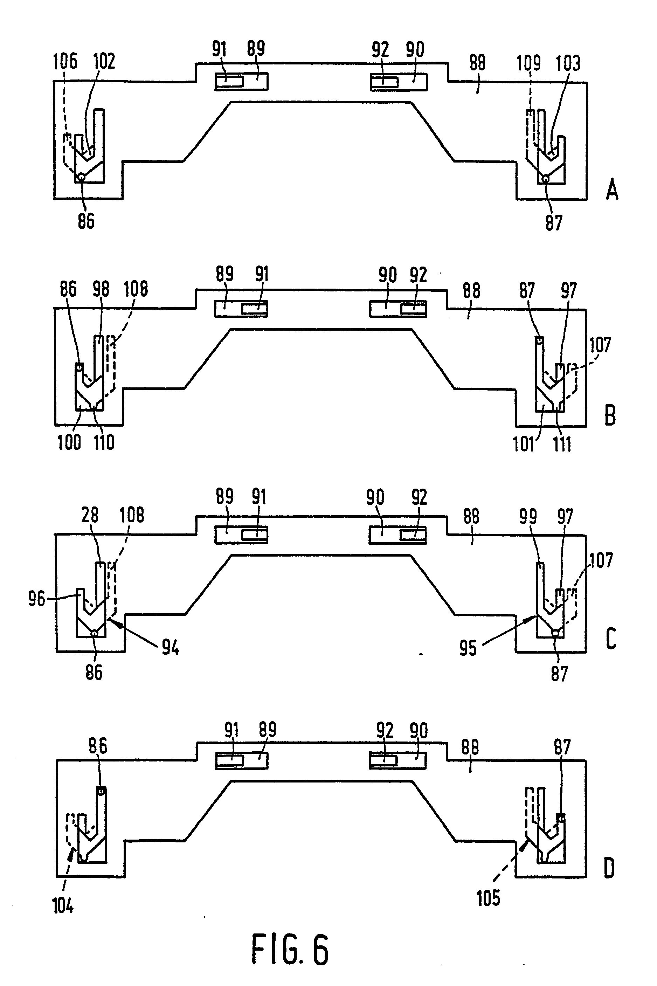

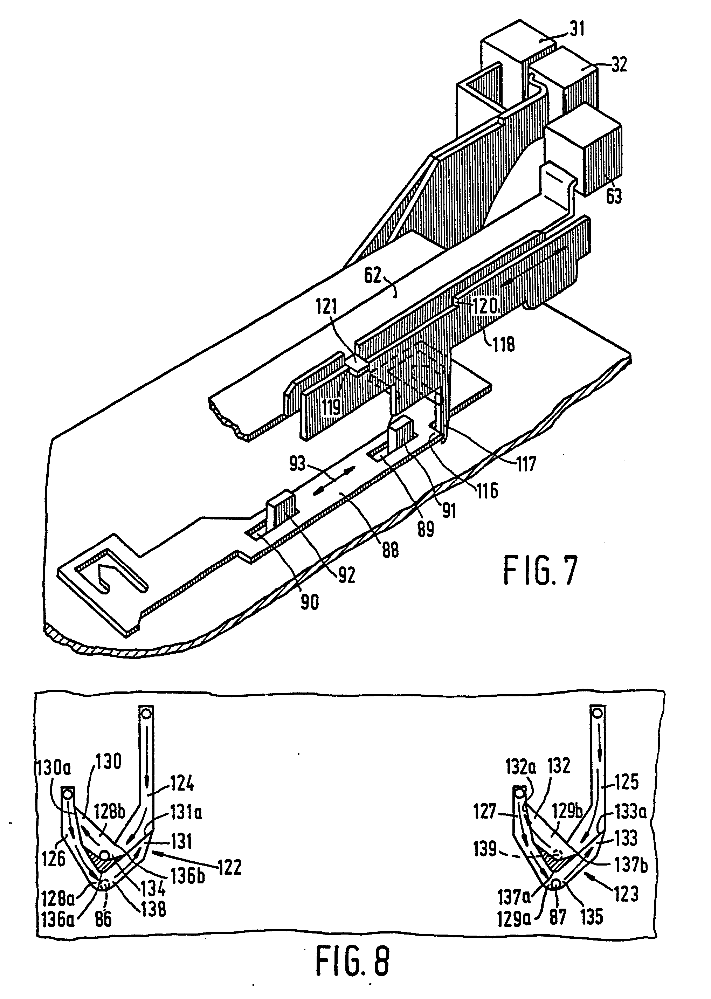

Fig. 7 zeigt anhand einer schaubildlichen Darstellung auf der Chassisplatte des Gestells 1 die Steuerplatte 88. Die Steuerplatte 88 ist mit einem Anschlag 116 versehen, gegen den eine Nase 117 eines Zwischenschiebers 118 stoßen kann, der parallel zu der gedachten Verbindungslinie a zwischen den Tonwellen 9 und 10 verschiebbar ist. Der Zwischenschieber 118 ist mit zwei Anschlägen 119, 120 versehen, zwischen die ein Mitnehmer 121 der auch als Auswerfer dienenden Stoptastenstange 62 drücken kann. Wenn die Stoptaste 63 mit ihrer Stange 62 ausgerückt ist, dann hat sie den Zwischenschieber 118 nach außen mitgenommen, weil der Mitnehmer 121 gegen den Anschlag 120 gestoßen ist. Dadurch ist die Nase 117 von dem Anschlag 116 abgerückt. Die Steuerplatte 88 ist damit ungehindert in Richtung des Doppelpfeiles 93 nach Fig. 5 und 7 verschiebbar. Wird die Stop/Auswurftaste 63 demgegenüber eingedrückt, dann stößt der Mitnehmer 121 gegen den Anschlag 119 und die Nase 117 gegen den Anschlag 116. Die Steuerplatte 88 ist damit in einer Vorzugslage nach Fig. 6B festgelegt.

Die Funktionsweise der Vorrichtung läßt sich wie folgt beschreiben: Wird eine Kassette eingelegt, dann fährt die Auswurftaste 63 aus der Stellung nach Fig. 2A in die Stellung nach Fig. 2B aus. Auf ebenfalls nicht dargestellte Weise wird der Schalter des Motors 3 geschlossen, und der Motor beginnt zu drehen. Damit drehen sich auch die Schwungscheiben 7 und 8, und zwar in gegensinniger Richtung. Gleichzeitig drehen sich die Zahnräder 11 und 12 und die mit ihnen kämmenden Zahnräder 14 und 17.

Durch das Ausfahren der Auswurfstange 62 schwenkt der Rasthebel 58 nach Fig. 2A um die Achse 59, und der Raststift 65 kommt in den Wirkbereich mit der Herzkurve 56 nach Fig. 2B.

Weil die Zahnräder 14 und 17 nicht in Einriff sind mit den Zahnrädern 24 und 25, drehen die beiden Wickeldorne 20 und 21 nicht mit. Die Scheiben 41 und 42 sind über ihre separaten Reibungskupplungen mit àen Wickeldornen 20, 21 verbunden und stehen damit auch still. Da beim Eindringen der Kassette ein Dremoment auf das Schaltelement 46 fehlt, weil die Bandwickel nicht umlaufen und das Band still steht, bleibt das Schaltelement 46 in der in Fig. 1 dargestellten Stellung stehen und kommt damit mit dem Stift 49 auf die Außenseite der Steuerkurve 41. Damit macht das Schaltelement 46 eine Bewegung im Uhrzeigersinn gemäß Pfeil 46a und schwenkt das Verbindungselement 52 entgegen dem Uhrzeigersinn (Pfeil 67). Weil der Rasthebel 58 geschwenkt ist und der Stift 65 der Herzkurve 56 im Wirkbereich ist, umläuft der Stift 65 nun die Wand 68 der Herzkurve 56 und fällt in die Mulde 70 ein. Damit ist das Verbindungselement in einer eingeschwenkten Stellung eingerastet (siehe Fig. 2B).

Durch das Schwenken des Verbindungselementes 52 entgegen dem Uhrzeigersinn in Richtung des Pfeiles 67 hat sich der Nocken 71 nach innen bewegt und gegen die Blattfeder 72 gedrückt. Die Blattfeder drückt ihrerseits über die Halterungen 76 und 77 gegen die Kopfplatte 74 und versucht, sie in Richtung auf die Tonwellen 9, 10 zu schieben. Nun kommt die Wirkung der Steuerplatte 88 zur Geltung.

Vor dem Einschwenken des Nockens 71 war die Kopfplatte 74 von den Tonwellen weggefahren. Die Leitstifte 86, 87 waren dadurch in die Einlaufschlitze 110 und 111 eingelaufen (vergl. Fig. 5). Die Steuerplatte 88 hat die aus Fig. 6A zu ersehende angenommene Stellung. Beim Vorfahren der Kopfplatte 74 in Richtung der Tonwellen 9, 10 fahren die Leitstifte 86, 87 nach vorn und laufen auf der linken Seite der Zungen 102, 103 auf und zugleich in die linken Schenkel 106 und 109 der gabelförmigen Leitschlitze 104, 105. Bei dem Einfahren der Leitstifte 86, 87 in die Schenkel 106 und 109 nehmen die Leitstifte 86, 87 die Steuerplatte 88 nach links mit, so daß sich nunmehr die Schenkel 96, 106 und 99, 109 überdecken. Der linke Leitstift 86 findet damit in dem kürzeren Schenkel beim Vorfahren einen Anschlag, während der rechte Leitstift 87 frei in Richtung auf die Tonwelle 10 vorlaufen kann (Fig. 6B). Damit schwenkt die vorgedrückte Kopfplatte 74 auf der rechten Seite derartig nach vorn, daß die rechte Andruckrolle 79 gegen die rechte Tonwelle 10 stößt. Die andere Tonwelle bleibt unbelastet. Damit läuft das Band in der aus Fig. 3 zu ersehenden Richtung.

Beim Erreichen des Bandendes bleibt das Band stehen. Dadurch wird das Schaltelement 46 mit seinem Stift 49 erneut nach außen im Uhrzeigersinn geschwenkt. Dadurch wird das Verbindungselement 52 erneut entgegen dem Uhrzeigersinn verschwenkt (Fig. 2B) in Richtung des Pfeiles 67. Das Verbindungselement 52 wird bei diesem Bewegen über den Normalhub, der bei der eingerasteten Lage vorliegt, in einem Überhub weitergeschwenkt. Der Überhub wird möglich, da der Nocken 71 gegen die Blattfeder 72 über die Hebel 76, 77 an der Kopfplatte 74 drückt und damit Ausweichspielraum in Richtung auf die Kopfplatte hat. Bei diesem Überhub fährt der Stift 65 aus der Mulde 70 heraus, und der Rastmechanismus 57 entrastet. Unter Wirkung der Feder 54 schwenkt das entrastete Verbindungselement 52 nun im Uhrzeigersinn, so daß der Nocken 71 nach außen fährt. In dieser Lage ist die Kopfplatte nach außen zurückgefahren, und die Leitstifte 86, 87 sind wieder in die Einlaufschlitze 110 und 111 eingelaufen. Die Steuerplatte 88 ist in ihrer alten Stellung verblieben. Die Zungen 102 und 103 liegen damit noch immer in einer nach links verschobenen Lage gegenüber der mittigen Lage der Einlaufschlitze 110 und 111 Fig. 6C).

Es kommt nun der Reversiervorgang. Das Schaltrad 14 dreht nach wie vor weiter. Da sich die Kopfplatte 74 zurückgezogen hat, hat sie ihr umgebogenes Ende 80 auch nach außen gezogen und damit den Schwenkarm 16 derart verschwenkt, daß das Schaltrad 14 außer Eingriff ist vom Spielrad 25. Damit wird das Spielrad 25 nicht mehr angetrieben. Das Detektionsorgan 40 stellt Bandstillstand fest. Aufs Neue wird das Schaltelement 46 im Uhrzeigersinn verschwenkt. Das Schaltelement 46 drückt gegen das Verbindungselement 52 und schwenkt dieses erneut entgegen dem Uhrzeigersinn in Richtung des Pfeiles 67. Damit umfährt der Stift 65 erneut die Herzkurve 56, und der Stift fällt letztlich wieder in die Mulde 70 ein, und das Verbindungselement 52 verrastet. Der Nocken 71 ist nun wieder nach vorn geschoben, und die Kopfplatte 74 nimmt nach ihrem Vorfahren die aus Fig. 4 ersichtliche Stellung ein. Das Zahnrad 17 ist über das umgebogene Ende 81 der Kopfplatte 74 mit dem Zahnrad 11 und mit dem Spielrad 24 im Eingriff. In dieser Lage nach Fig. 4 liegt die Andruckrolle 78 an der Tonwelle 9 an, und das Band wird in entgegengesetzter Richtung transportiert.

Die Umkehrung der Bandtransportrichtung ist wieder durch die Leitschlitzanordnung bewirkt worden, die in Fig. 6 in verschiedenen Stellungen dargestellt ist. Die letzte beschriebene Stellung war die Stellung nach Fig. 6C. In dieser Stellung ist die Steuerplatte 88 nach links verschoben, und die Leitstifte 86, 87 liegen in den Einlaufschlitzen 110 und 111. Durch das erneute Vorfahren der Kopfplatte 74 sind die Leitstifte 86, 87 ebenfalls nach vorn gefahren und sind gegen die rechten Schrägflächen der angespitzten Zungen 102 und 103 gelaufen. Die Leitstifte 86, 87 wurden dadurch nach rechts verschoben und konnten weiter einfahren in die Schenkel 108 und 107. Die Querverschiebung der Leitstifte 88, 87 ist möglich durch das Verrutschen der Leitstifte 86, 87 in den Längsschlitzen 84, 85. Bei dem Einfahren in die Schenkel 107 und 108 haben die Leitstifte 86, 87 die Steuerplatte 88 nach rechts mitgenommen, so daß sich nunmehr die Schenkel 108 und 98 sowie 107 und 97 überdecken. Damit haben sich die Zungen 102, 103 wieder so versetzt, daß bei einem erneuten Zurückfahren und Vorfahren der Leitstifte diese dann wieder auf die linken Schrägflächen der angespitzten Zungen 102 und 103 auflaufen können. Die Stellung der Leitschlitzanordnung in diesem Fall ist aus Fig. 6D zu ersehen. Der Leitstift 87 hat in den kürzeren Schenkeln 107 und 97 auf seinem Vorfahrweg einen Anschlag vorgefunden, der die Kopfplatte 74 daran hindert, auf der rechten Seite weiter vorzufahren. Auf der linken Seite hat der Leitstift 86 keinen Anschlag vorgefunden und er konnte in den Schenkeln 108 und 98 so weit vorfahren, daß die Feder 72 die linke Seite der Kopfplatte 74 die Andruckrolle 78 gegen die Tonwelle 9 drücken konnte. Diese Stellung ist aus Fig. 4 zu ersehen.

In Fig. 8 ist eine abgewandelte Ausführungsform der Leitschlitzanordnung und der Steuermittel dargestellt. Die Leitschlitzanordnung besteht aus U-förmigen Rinnen 122, 123, die an der Chassisplatte des Gestells 1 vorgesehen sind. Die U-förmigen Rinnen wirken wieder mit den Leitstiften 86, 87 zusammen. Die U-förmigen Rinnen 122 und 123 haben, wie bei der Ausführungsform nach den Fig. 5 und 6, lange Schenkel 124, 125 und kurze Schenkel 126, 127. Die verbindende Basis zwischen den jeweiligen Schenkeln ist als Doppelrinne 128a, 128b und 129a, 129b ausgebildet. Im Bereich dieser Doppelrinnen 128 und 129 sind als Steuermittel wirkende Führungsflächen 130 und 131 sowie 132 und 133 vorgesehen. Die Führungsflächen 130 bis 133 sind als schräge Auflaufflächen ausgebildet, die sich aus dem Rinnenboden langsam erheben, bis sie an Leitkonturen 130a, 131a, 132a und 133a enden. Diese Führungskonturen gehören ebenfalls zu den Steuermitteln für die Leitstifte 86 und 87.

Die Funktion der Leitschlitzanordnungen und Steuermittel nach Fig. 8 lassen sich wie folgt beschreiben: Es sei angenommen, daß die Kopfplatte 74 zurückgezogen ist. In dieser Ausgangslage liegt der Leitstift 86 in einer Mulde 134 der oberen Doppelrinne 128b. Der Leitstift 87 liegt in einer Mulde 135 der unteren Doppelrinne 129a. Oberhalb der Leitstifte sind Rinnenwände mit Leitpunkten 137a und 136b versehen. Diese Leitpunkte sind gegenüber den Mitten der Leitstifte 86, 87 an der unteren Rinne nach links und an der oberen Rinne nach rechts versetzt. Damit können die Leitstifte, wenn die Kopfplatte 74 erneut eingedrückt wird, in der oberen Rinne nur nach links auf die Führungsfläche 130 und aus der unteren Rinne nur in Richtung auf die Führungsfläche 133 nach rechts vorfahren. Das bedeutet, daß bei diesem Vorfahrvorgang der Leitstift 86 in den kürzeren Schenkel 126 und der Leitstift 87 in den längeren Schenkel 125 einfährt. Der Leitstift 86 erfährt also beim Vorfahren nach einem kürzeren Vorfahrweg einen Anschlag, während der Leitstift 87 frei vorfahren kann. Das entspricht der Stellung der Kopfplatte 74 nach Fig. 3. Wird die Kopfplatte 74 erneut zurückgefahren, dann läuft der Leitstift 86 durch den Schenkel 126 in die untere Rinne 128a und der Leitstift 87 in die obere Rinne 129b ein. In der ganz zurückgefahrenen Stellung liegt damit der Leitstift 86 in einer Mulde 139 (gestrichelte Lage in Fig. 8) und der Leitstift 87 in einer Mulde 139. Wird die Kopfplatte nun erneut in Richtung auf die Tonwellen vorgefahren, dann läuft der Leitstift 86 in Richtung auf die Führungsfläche 131 in den langen Schenkel 124 ein. Der Leitstift 87 läuft in Richtung auf die Führungsfläche 132 in den kurzen Schenkel 127 ein. Das bedeutet, daß der Leitstift 87 in diesem Fall in den kurzen Schenkel 127 einen verkürzten Vorfahrweg mit einem Anschlag vorfindet, während der Leitstift 86 frei in dem Schenkel 124 vorfahren kann. Die Kopfplatte erfährt damit eine Stellung, die aus Fig. 4 zu ersehen ist. Beim erneuten Zurückfahren der Kopfplatte 84 laufen die Leitstifte 86 und 87 wieder in die zuerst beschriebene Ausgangslage ein.

Der Vorteil dieser Leitschlitzanordnung besteht darin, daß keine beweglichen Teile zum Steuern der Verfahrbahnen der Leitstifte erforderlich sind. Die Rinnen und Führunsflächen sowie Führungskonturen lassen sich an der Chassisplatte in Kunststoff spritzen.

**Patentansprüche**

1. Vorrichtung an dem reversierenden Laufwerk eines Magnetbandkassettengerätes, bei dem das Magnetband an wenigstens einem Aufnahme- bzw. Wiedergabetonkopf (75) vorbeigeführt wird, der an einer als Schwinge ausgebildeten, gegen das Magnetband verfahrbaren Kopfplatten (74) angeordnet ist, die infolge eines gesteuerten Verschwenkens immer eine von zwei an ihr angeordneten Andruckrollen (78, 79) gegen die zugehörige Tonwelle (9, 10) überführt und damit die Bandlaufrichtung bestimmt, gekennzeichnet durch
- am Laufwerkschassis (1) vorgesehene, beiderseits des Tonkopfes (75) angeordnete Leitschlitzanordnungen, (104, 105),
- an der Kopfplatte (74) vorgesehene Leitstifte (86, 87), die in den Leitschlitzanordnungen auf die gedachte Tonwellen-Verbindungslinie (a) zufahren können,
- mit den Leitschlitzanordnungen zusammenwirkende Steuermittel (88; 130 - 133; 130a, 133a), die die Leitstifte (86, 87) in den Leitschlitzanordnungen derart lenken, daß die Leitstifte (86, 87) nach jedem Zurückfahren der Kopfplatte (74) von der Verbindungslinie (a) beim erneuten Vorfahren auf die Verbindungslinie (a) wechselweise in der einen oder anderen Leitschlitzanordnung in einen Leitschlitzschenkel (96 - 99; 106 - 109; 124 - 127) einfahren, der dem betreffenden Leitstift (86, 87) den Vorfahrweg blockiert, wodurch jeweils die Andruckrolle (78, 79) der verschwenkenden Kopfplatte (74), deren zugeordneter Leitstift (86, 87) einen unblockierten Vorfahrweg vorfindet, zur zugeordneten Tonwelle (9, 10) vorfahren kann.

2. Vorrichtung nach Anspruch 1, dadurch gekennzeichnet, daß die Leitschlitzanordnungen aus am Laufwerkschassis (1) vorgesehenen gabelförmigen Leitschlitzen (104, 105) mit zwei den Gabelzinken entsprechenden Schenkeln (106, 108 und 107, 109) bestehen, von denen einer kürzer als der andere ausgebildet ist und die kürzeren (bzw. längeren) Schenkel (106, 107) beide entweder aufeinander zu oder voneinander weg angeordnet sind, und daß sich ein dem Gabelschaft entsprechender mittiger Einlaufschlitz (110, 111) an den trichterförmigen Gabelfuß (112, 113) anschließt.

3. Vorrichtung nach den Ansprüchen 1 und 2, dadurch gekennzeichnet, daß die Steuermittel aus einer Steuerplatte (88) bestehen, die parallel zur Tonwellen-Verbindungslinie (a) zwischen zwei Endstellungen verschiebbar und beiderseits des Tonkopfes (75) mit U-förmigen Leitschlitzen (94, 95) die zugeordneten gabelförmigen Leitschlitze (104, 105) überdeckbar sind, jedoch einen geringeren gegenseitigen Abstand (114, 115) voneinander aufweisen, wodurch zum wechselweisen Positionieren der Leitstifte (86, 87) in die kürzeren oder längeren Schenkel die angespitzten freien Enden der Materialzungen (102, 103) zwischen den Schenkeln der U-förmigen Leitschlitze (94, 95) - in den Steuerplattenendstellungen und bei zurückgefahrener Kopfplatte (74) - wechselweise in der einen oder anderen Verschieberichtung seitlich gegenüber den Einlaufschlitzen (110, 111) versetzbar sind.

4. Vorrichtung nach Anspruch 1, dadurch gekennzeichnet, daß die Leitschlitzanordnungen aus am Laufwerkschassis (1) vorgesehenen U-förmigen Rinnen (122, 123) mit jeweils einem kürzeren (126, 127) und einem längeren (124, 125) Schenkel und einer Doppelrinne (128a, b und 129a, b) an der die Schenkel (124 - 127) verbindenden Basis versehen sind und daß die Steuermittel als erhabene Führungsflächen (130 - 133) am Rinnenboden und Führungskonturen (130a-133a) im Bereich des Rinnenbodens ausgebildet sind, die es den Leitstiften (86, 87) nach jedem Zurückfahren mit der Kopfplatte (74) und beim erneuten Vorfahren nur gestatten, über eine der Basisrinnen (128a, b und 129a, b) von dem kürzeren der Schenkel (126, 127) in den längeren Schenkel (124, 125) und beim nachfolgenden Zurück- und Vorfahren über die andere Basisrinne (128a, b und 129a, b) von dem längeren in den kürzeren Schenkel einzulaufen, wobei die kürzeren und längeren Schenkel so verteilt sind, daß immer bei einem Zurück- und Vorfahren einer der Leitstifte (86, 87) in einen kürzeren Schenkel einläuft, während der andere Leitstift (86, 87) in den längeren Schenkel einläuft, und beim darauffolgenden Zurück- und Vorfahren dieser Leitstift (86, 87) in den längeren und der andere in den kürzeren Schenkel einläuft.

5. Vorrichtung nach den Ansprüchen 1 bis 4, dadurch gekennzeichnet, daß die Leitstifte (86, 87) in der Kopfplatte (74) in parallel zur Tonwellen-Verbindungslinie (a) verlaufenden Längsschlitzen (84, 85) verschiebbar geführt sind.

6. Vorrichtung nach Anspruch 3, dadurch gekennzeichnet, daß die Steuerplatte (88) mit wenigstens einem Anschlag (116) versehen ist und daß das Gerät eine Stoptaste (63) und eine damit verbundene Stange (62) aufweist, die mit einer Nase (117) versehen ist, die beim Bedienen der Stoptaste (63) gegen den Anschlag (116) stößt und die Steuerplatte (88) immer in die eine ihrer Endstellungen verschiebt.

## Claims

1. A device in the auto-reverse deck of a magnetic-tape-cassette apparatus, in which the magnetic tape is passed along at least one magnetic recording playback head (75) which is arranged on a pivotable head-mounting plate (74) which is movable towards the magnetic tape and which by a controlled pivotal movement positions always one of the two pressure rollers (78, 79) arranged on it against the associated capstan (9, 10) and thereby defines the direction of tape transport, characterized by
- guide slot systems (104, 105) arranged on the tape-deck chassis (1) on both sides of the magnetic head (75),
- guide pins (86, 87) which are arranged on the head-mounting plate (74) and which can move in the guide slot systems towards an imaginary capstan connecting line (a),
- control means (88; 130-133; 103a-133a) which cooperate with the guide-slot systems and which guide the guide pins (86, 87) in the guide-slot systems in such a way that, after each movement of the head-mounting plate (74) away from the connecting line (a), during a subsequent movement towards the connecting line (a) the guide pins (86, 87) alternately move in the one or the other guide-slot system into a guide slot branch (96-99; 106-109; 124-127) which blocks the travel of the relevant guide pin (86, 87), so that that pressure roller (78, 79) on the pivoting head-mounting plate (74) can move towards the associated capstan (9, 10) whose associated guide pin (86, 87) meets a path which is not blocked.

2. A device as claimed in Claim 1, characterized in that the guide-slot systems comprise forkshaped guide slots (104, 105) in the tape-deck chassis (1), which slots comprise two branches (106, 108 and 107, 109) corresponding to the fork tines, one of the branches being shorter than the other and the shorter (or longer) branches (106, 107) both being situated either nearer one another or remote from each other, and in that a central run-in branch (110, 111) which corresponds to the fork handle opens into the trough-shaped fork base (112, 113).

3. A device as claimed in Claims 1 and 2, characterized in that the control means comprise a control plate (88) which can be moved parallel to the capstan connecting-line (a) between two end positions and in which U-shaped guide slots (94, 95) are formed on both sides of the sound head (75), which slots can be made to coincide with the associated forks-shaped guide slots (104, 105) but which have a smaller distance from each other, so that for alternately positioning the guide pins (86, 87) in the short or the long branches the pointed free ends of the tongues (102, 103) between the branches of the U-shaped guide slots (94, 95) - in the control plate end positions and with refracted head-mounting plate - can alternately be offset in a lateral direction relative to the run-in branches (110, 111) in the one or the other direction of the movement.

4. A device as claimed in Claim 1, characterized in that the guide-slot systems comprise U-shaped grooves (122, 123) formed in the tape-deck chassis (1), which grooves each have a short (126, 127) and a long branch (124, 125) and a double groove (128a, b and 129a, b) at the base connecting the branches (124-127), and in that the control means comprise raised guide surfaces (130-133) on the groove bottoms and guide portions (130a-133a) in the area of the groove bottom, which after each withdrawal and subsequent forward movement of the headmounting plate (74) only allow the guide pins (86, 87) to move via one of the base grooves (128a, b and 129a, b) from the short branch (126, 127) into the long branch (124, 125) and during the next backward and forward movement to move via the other base groove (128a, b and 129a, b) from the long into the short branch, the short and the long branches being so arranged that upon each backward and forward movement one of the guide pins (86, 87) moves into a short branch, whilst the other guide pin (86, 87) moves into the long branch, and during the subsequent backward and forward movement said guide pin (86, 87) moves into the long and the other into the short branch.

5. A device as claimed in any one of the Claims 1 to 4, characterized in that the guide pins (86, 87) are guided in longitudinal slots (84, 85) in the head-mounting plate (74) so as to be movable parallel to the capstan connecting line (a).

6. A device as claimed in Claim 3, characterized in that the control plate (88) is provided with at least one stop (116) and in that the apparatus comprises a stop button (63) and a rod (62) which is connected to said button, which rod is provided with a projection (117) which upon actuation of the stop button (63) abuts against the stop (116) and always moves the control plate (88) into one of its end positions.

## Revendications

1. Dispositif faisant partie du mécanisme d'entraînement réversible d'un appareil à cassette de bande magnétique, dans lequel la bande magnétique défile en regard d'au moins une tête magnétique d'enregistrement/lecture (75) montée sur une plaque porte-tête (74) ayant la forme d'un élément oscillant et déplaçable en direction de la bande magnétique, qui, suite à un pivotement commandé, amène toujours l'un de deux galets presseurs (78, 79) qu'il porte contre le cabestan associé (9, 10) et détermine ainsi le sens de défilement de la bande, caractérisé par:
- des ensembles de fentes de guidage (104, 105) prévus sur le châssis (1) du mécanisme d'entraînement, de part et d'autre de la tête magnétique (75),
- des broches de guidage (86, 87) prévues sur la plaque porte-tête (74), qui, dans les ensembles de fentes de guidage, peuvent parvenir à la ligne imaginaire (a) reliant les cabestans,
- des moyens de commande (88; 130 - 133; 130a, 133a) coopérant avec les ensembles de fentes de guidage qui pilotent les broches de guidage (86, 87) dans les ensembles de fentes de guidage d'une manière telle que les broches de guidage (86, 87), après chaque recul de la plaque porte-tête (74) depuis la ligne de liaison (a), lors d'un nouvel avancement vers la ligne de liaison (a), s'engagent alternativement dans l'un ou l'autre des ensembles de fentes de guidage dans une branche de fente de guidage (96 - 99; 106 - 109; 124-127) qui bloque le trajet d'avancement de la broche de guidage (86, 87) en question, grâce à quoi le galet presseur (78, 79) de la plaque porte-tête (74) qui pivote, dont la broche de guidage (86, 87) associée rencontre un trajet d'avancement non bloqué, peut chaque fois avancer vers le cabestan (9, 10) associé.

2. Dispositif suivant la revendication 1, caractérisé en ce que les ensembles de.fentes de guidage sont constitués de fentes de guidage de forme fourchue (104, 105) prévues dans le châssis (1) du mécanisme d'entraînement et comportant deux branches (106, 108 et 107, 109) correspondant aux fourchons, dont l'une est plus courte que l'autre et les branches courtes (ou longues) (106, 107) des deux fentes sont rapprochées l'une de l'autre, respectivement écartées l'une de l'autre et, au niveau de l'enfourchure, une fente d'entrée médiane (110, 111) se raccorde à la base de fourches (112, 113) en forme d'entonnoirs.

3. Dispositif suivant les revendications 1 et 2, caractérisé en ce que les moyens de commande sont constitués d'une plaque de commande (88) qui peut se déplacer parallèlement à la ligne de liaison (a) des cabestans entre deux positions d'extrémité et est pourvue, de part et d'autre de la tête magnétique (75), de fentes de guidage en U (94, 95) dont les branches peuvent être couvertes par les branches des fentes de guidage en forme de fourches (104, 105) associées, mais présentent une plus courte distance réciproque (114, 115) entre elles, de sorte que, pour le positionnement alternatif des broches de guidage (86, 87) dans les branches courtes ou longues, les extrémités libres pointues des languettes de matière (102, 103) prévues entre les branches des fentes de guidage en U (94, 95) (dans les positions d'extrémité de la plaque de commande et après le recul de la plaque porte-tête) peuvent être décalées alternativement dans un sens de déplacement ou l'autre latéralement par rapport aux fentes d'entrée (110, 111).

4. Dispositif suivant la revendication 1, caractérisé en ce que les ensembles de fentes de guidage sont formés de rainures en forme de U (122, 123) prévues dans le châssis (1) du mécanisme d'entraînement et comportant chacune une branche courte (126, 127) et une branche longue (124, 125) ainsi qu'une double rainure (128a, b et 129a, b) au niveau de la base de liaison des branches (124 - 127) et que les moyens de commande ont la forme de surfaces de guidage (130 - 133) en saillie dans la zone du fond des rainures, et de contours de guidage (130a - 133a) dans la zone du fond des rainures, ce qui ne permet aux broches de guidage (86, 87), après chaque recul avec la plaque porte-tête (74) et lors d'un nouvel avancement, que de s'engager, par l'intermédiaire d'une rainure de base (128a, b et 129a, b) de la branche courte (126, 127), dans la branche longue (124, 125) et, lors d'un recul et d'un avancement suivant, de s'engager, par l'intermédiaire de l'autre rainure de base (128a, b et 129a, b) de la branche longue, dans la branche courte, les branches courtes et longues étant réparties d'une manière telle que, lors d'un recul et d'un avancement, une des broches de guidage (86, 87) s'engage toujours dans une branche courte, tandis que l'autre broche de guidage (86, 87) s'engage dans la branche longue et, lors du recul et de l'avancement qui suivent, cette broche de guidage (86, 87) s'engage dans la branche longue et l'autre dans la courte.

5. Dispositif suivant l'une quelconque des revendications 1 à 4, caractérisé en ce que les broches de guidage (86, 87) dans la plaque porte-tête (74) sont guidées de manière à pouvoir se déplacer dans des fentes longitudinales (84, 85) qui s'étendent parallèlement à la ligne (a) reliant les cabestans.

6. Dispositif suivant la revendication 3, caractérisé en ce que la plaque de commande (88) est pourvue d'au moins une butée (116) et que l'appareil comporte une touche d'arrêt (63) et une tige (62) reliée à celle-ci qui est pourvue d'un bec (117) qui lorsque la touche d'arrêt (63) est actionnée, bute contre la butée (116) et déplace la plaque de commande (88) toujours dans la première de ses positions d'extrémité.

FIG.1

FIG.2A

FIG.2B

FIG.3

FIG. 4

0 121 281

FIG. 5

FIG. 6

FIG. 7

FIG. 8